# EUROPEAN PATENT SPECIFICATION

(11) **EP 2 566 437 B1**
(45) Date of publication and mention of the grant of the patent: **20.07.2016**
(21) Application number: 11719805.1
(22) Date of filing: 06.05.2011
(51) Int. Cl.: A61K 8/365, A61K 8/58, A61K 8/06, A61Q 19/00, A61K 8/898, A61K 8/891, A61K 8/25

(54) **HIGH SOLVENT CONTENT EMULSIONS**
EMULSION MIT EINEM HOHEN GEHALT AN LÖSUNGSMITTEL
ÉMULSIONS À TENEUR ÉLEVÉE EN SOLVANT

(30) Priority: 07.05.2010 WO PCT/CN2010/000639
(43) Date of publication of application: 13.03.2013
(73) Proprietor: Unilever PLC, London, Greater London EC4Y 0DY (GB); Unilever N.V., 3013 AL Rotterdam (NL)
(72) Inventor: ZHANG, Qiqing, Shanghai 200335 (CN); KHOSHDEL, Ezat, Wirral Merseyside CH63 3JW (GB); MOADDEL, Teanoosh, Trumbull, CT 06611 (US); DOBKOWSKI, Brian, John, Trumbull, CT 06611 (US); ZHOU, Weizheng, Shanghai 200335 (CN); YUAN, Su, Shanghai 200335 (CN)
(74) Representative: Corsten, Michael Allan
(86) International application number: PCT/EP2011/057333
(87) International publication number: WO 2011/138445

(56) References cited:
- US-A- 5 106 609
- US-A1- 2002 054 890
- US-A1- 2007 243 220

## Description

### FIELD OF THE INVENTION

The present invention is directed to a high solvent content emulsion. More particularly, the present invention is directed to an emulsion comprising at least 20% by weight solvent, functionalized polymer and particles having an isoelectric point below the pH of the emulsion whereby the emulsion is suitable for use as a cosmetically acceptable vehicle that unexpectedly yields excellent sensory benefits.

### BACKGROUND OF THE INVENTION

Emulsions are typically defined to mean heterogeneous systems which have two immiscible liquids or phases. One liquid is in dispersed form while the other forms droplets within the dispersed liquids. Emulsions can be employed as carriers for actives, especially those targeted for application to skin.

It is customary to use emulsifiers in order to achieve permanent dispersion of one liquid in another. While emulsifiers are customarily used in vehicles for topical compositions, emulsifiers are linked to both allergic and non-allergic reactions of consumers using such compositions. Moreover, use of customary emulsifiers can result in formulations that do not deliver excellent sensory benefits, are difficult to apply, and are generally not environmentally friendly.

Attempts at avoiding the need for traditional emulsifiers have been made. These attempts require the use of micro fine particles (like inorganic pigment) to produce what is known as a Pickering emulsion. Such emulsions are, however, known for their low water content and are not characteristically known to deliver excellent sensory and deliverability characteristics.

It is of increasing interest to develop emulsions comprising a high solvent content, and preferably, emulsions that are substantially free of emulsifier or surfactant. Particularly, it is desirable to develop emulsions that deliver excellent sensory benefits and that do not have a negative impact on the environment. This invention, therefore, is directed to an emulsion comprising at least 20% by weight solvent. The emulsion comprises a functionalized polymer and particles having an isoelectric point below the pH of the emulsion whereby the same delivers excellent sensory and active deliverability benefits when applied or used by a consumer even after being formulated substantially free of emulsifier or surfactant.

### ADDITIONAL INFORMATION

Efforts have been disclosed for making emulsions. In US 6 379 680, US 6 767 547 and US 6 838 088, Pickering emulsions are described.

Still other efforts have been disclosed for making emulsions. In US 5 910 467, solids-stabilized emulsions are described.

Even other efforts have been disclosed for making emulsions. In US 6 710 092, emulsions stabilized with polymer resin precursors are described.

None of the additional information above describes an emulsion comprising at least 20% by weight solvent, functionalized polymer and particles having an isoelectric point below the pH of the emulsion.

### SUMMARY OF THE INVENTION

In a first aspect, the present invention is directed to an emulsion comprising:
(a) particles having an isoelectric point below a pH of the emulsion;
(b) functionalized polymer suitable to hydrogen and/or ionically bond to the particles; and
(c) solvent.

The emulsion of the first aspect is substantially free of surfactant.

In a second aspect, the present invention is directed to an end use composition comprising the emulsion of the first aspect of this invention.

In a third aspect, the present invention is directed to a method for treating a condition by applying the composition of the second aspect of this invention.

High solvent content emulsion, as used herein, means an emulsion having at least 20% by weight solvent, the same suitable for use as a vehicle or carrier for an end use composition. A particle having an isoelectric point below a pH of the emulsion means a particle within the emulsion suitable to complement hydrogen and/or ionic bonding tendencies of the functionalized polymer. Typically, the particle has an isoelectric point under 11, and preferably, under 7.5. End use composition means a composition ready for use by a consumer and comprising the emulsion of this invention whereby the high solvent content emulsion of this invention is preferably a high (water-in-oil) internal phase emulsion which is preferably prepared substantially free of surfactant. Illustrative examples of the types of end use compositions that may comprise the high solvent content emulsion of this invention include leave-on skin lotions and creams, shampoos, hair conditioners, shower gels, toilet bars, antiperspirants, deodorants, shaving creams, depilatories, lipsticks, foundations, mascara, sunless tanners, and sunscreen lotions. End use compositions made with the high solvent content emulsion of this invention may also include a product associated with oral, laundry or household care such as laundry detergents, fabric conditioners or hard surface cleaners. In a preferred embodiment, however, the end use composition of this invention is applied to hair, skin or both, and most preferably skin, where skin is meant to include skin on the face, body and scalp. Deliverability means suitable to carry an active to a surface like a body surface through topical application.

Active means a component typically delivered to a surface including a body surface of an animal or human, preferably human, in order to enhance/improve a characteristic (e.g. appearance, cleanliness, odor) of the surface. Such an active can include, for example, a skin whitening or lightening ingredient like niacinamide as well as a hair conditioning agent and moisturizing agent like glycerine as well as those generally classified as silicon copolymers and quaternary ammonium salts. Functionalized polymer suitable to hydrogen and/or ionically bond means a polymer able to non-covalently interact with a particle in a donor and/or acceptor capacity. Substantially free of means less than 3%, and preferably, less than 1.5%, and most preferably 0.0% by weight, based on total weight of the emulsion. Excellent sensory benefits means yielding a silky sensation.

Comprising, as used herein, is meant to include consisting essentially of and consisting of. All ranges identified herein are meant to include, implicitly, all ranges subsumed therein if reference to the same is not explicitly made.

### DETAILED DESCRIPTION OF THE PREFERRED EMBODIMENTS

The only limitations with respect to the type of particle that may be used in this invention is that the same can be employed in a composition suitable for use by consumers and is able to complement the hydrogen and/or ionic bonding capabilities of the functionalized polymer used herein.

In a preferred embodiment, particle used in this invention comprises at least 0.1 % by weight silicon dioxide (i.e. silica), preferably at least 25% by weight silicon dioxide, and most preferably at least 50% to 100% by weight silicon dioxide based on total weight of particle in the emulsion. The particle comprises pyrogenically produced silica comprising the following group:

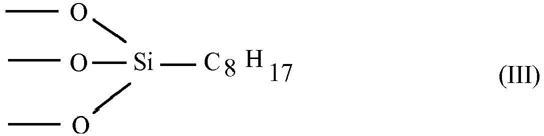

Silica of the octylsilane type and comprising the group represented by formula III is sold under the name Aerosil R805.

The high solvent content emulsion of the present invention often comprises from 0.25 to 45%, preferably from 1 to 25%, and most preferably from 1.5 to 15% by weight particle based on total weight of the emulsion and including all ranges subsumed therein.

The particle used typically has a diameter of less than three microns, preferably less than two microns, and most preferably from 10 nm to 1.5 microns including all ranges subsumed therein.

The functionalized polymers are of the amodimethicone type having the general formula: 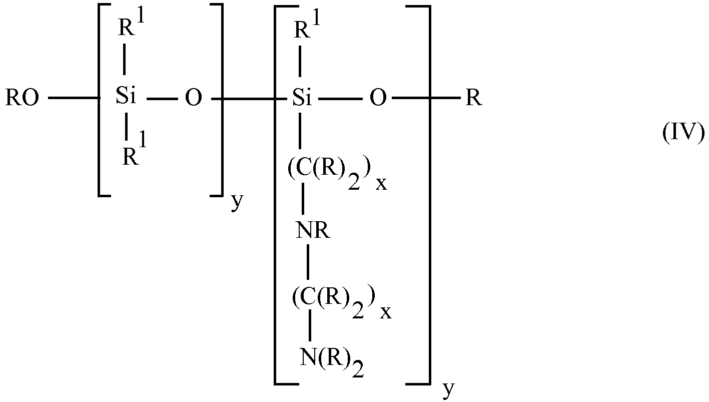 where:
each R is independently H, or a C₁₋₄ alkyl;
each R¹ is independently OR or a C₁₋₄ alkyl; and
each x is independently an integer from 1 to 4 and each y is greater than zero and independently an integer to yield a polymer having a molecular weight from 500 to 1 million, and preferably, from 750 to 25,000, and most preferably from 1,000 to 15,000.

Typically, the functionalized polymers make up from 0.1 to 75%, preferably from 0.3 to 50%, and most preferably from 0.75 to 20% by weight of the high solvent content emulsion, including all ranges subsumed therein. The used functionalized polymer is amodimethicone represented by formula (IV) and made commercially available, for example, as ADM 656 by Wacker-Belsil^{®} and DC 8500 by Dow Corning.

The solvent suitable for use in this invention is one which may be used in an end use product targeted to provide a benefit to consumers and one which is preferably capable of hydrogen bonding. The solvent used is water.

Solvent typically makes up from 20 to 85%, preferably from 30 to 75%, and most preferably from 40 to 70% by weight of the high solvent content emulsion, including all ranges subsumed therein.

Oils and/or fatty acids may optionally be mixed with the functionalized polymer to make the desired emulsion. Illustrative yet non-limiting examples of the types of oils that may be used in the emulsion generation include cyclic siloxanes like D5, as well as alkyl substituted and unsubstituted dimethicones. Especially preferred are C₂₆-C₂₈ alkyl dimethicones, lauryl dimethicone, mixtures thereof or the like where the same are commercially available from suppliers like Wacker-Belsil. Fatty acids preferred for use include C₈-C₂₀ fatty acids, especially caprylic or octanoic acid. When such oils or fatty acids are used in the generation of emulsion, they typically make up from 3 to 85%, preferably from 4 to 75%, and most preferably from 4 to 65% by weight of the total weight of mixture of oil and/or fatty acid, and functionalized polymer in the emulsion, including all ranges subsumed therein.

End use compositions comprising the high solvent content emulsion of this invention may optionally comprise co-vehicles or carriers like emollients, fatty acids, fatty alcohols, thickeners or combinations thereof.

Emollient materials may serve as optional cosmetically acceptable co-carriers along with the high solvent content emulsion of this invention. These may be in the form of silicone oils, natural or synthetic esters and hydrocarbons. Amounts of the emollients when used in addition to the high solvent content emulsion of this invention may range anywhere from 0.0 to about 30%, preferably between 1 and 25% by weight of the end use composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms. DC 245, made commercially available by Dow Corning, is often preferred.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethylsiloxanes with viscosities of from about 5 x 10⁻⁶ to 0.1 m²/s at 25°C. Among the preferred non-volatile emollients useful in the present end use composition are the polydimethyl siloxanes having viscosities from about 1 x 10⁻⁵ to about 4 x 10⁻⁴ m²/s at 25°C.

Another class of non-volatile silicones are emulsifying and non-emulsifying silicone elastomers. Representative of this category is Dimethicone/Vinyl Dimethicone Crosspolymer available as Dow Corning 9040 and Shin-Etsu KSG-18. Emulsifying silicone waxes such as Silwax WS-L (Dimethicone Copolyol Laurate) may also be useful. Other silicones suitable for use are blends sold as Dow Corning 9045.

Among the ester emollients are:
a) Alkyl esters of saturated fatty acids having 10 to 24 carbon atoms. Examples thereof include behenyl neopentanoate, isononyl isonanonoate, isopropyl myristate, isopropyl palmitate and octyl stearate.
b) Ether-esters such as fatty acid esters of ethoxylated saturated fatty alcohols.
c) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
d) Wax esters such as beeswax, spermaceti wax and tribehenin wax.
e) Sugar ester of fatty acids such as sucrose polybehenate and sucrose polycottonseedate.

Natural ester emollients principally are based upon mono-, di- and tri- glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may optionally range from about 0.1 to about 20% by weight of the end use compositions.

Hydrocarbons can optionally be employed as acceptable co-carriers with the high solvent content emulsions of this invention and they include petrolatum, mineral oil, C₁₁-C₁₃ isoparaffins, polybutenes, and especially isohexadecane, available commercially as Permethyl 101 A from Presperse Inc.

Fatty acids having up to 30 carbon atoms may also be employed as cosmetically acceptable co-carriers. Illustrative of this category are pelargonic, lauric, myristic, palmitic, stearic, isostearic, oleic, linoleic, linolenic, hydroxystearic and behenic acids.

Fatty alcohols having from 10 to 30 carbon atoms are another useful category of cosmetically acceptable carrier. Illustrative of this category are stearyl alcohol, lauryl alcohol, myristyl alcohol, oleyl alcohol and cetyl alcohol.

Thickeners can be utilized along with the high solvent content emulsion of this invention in the end use compositions according to the present invention. Typical thickeners include crosslinked acrylates (e.g., Carbopol 982^{®} and Carbopol Ultrez 21), hydrophobically-modified acrylates (e.g. Carbopol 1382®), polyacrylamides (e.g. Sepigel 305®), acryloylmethylpropane sulfonic acid/salt polymers and copolymers (e.g. Aristoflex HMB® and AVC®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, talc, calcium carbonate and silicates such as magnesium aluminum silicate (Veegum®). Amounts of the thickener may optionally range from 0.0001 to 10%, usually from 0.001 to 1%, optimally from 0.01 to 0.5% by weight of the end use composition.

Adjunct humectants may be employed in the end use compositions of the present invention. These are generally polyhydric alcohol-type materials. Typical polyhydric alcohols include glycerol, propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. If used, the amount of adjunct humectant may range anywhere from 0.5 to 20%, preferably between 1 and 15% by weight of the end use composition.

Surfactants, while not required or desired to prepare or make the high solvent content emulsion of this invention, may be present in end use compositions comprising the high solvent content emulsion of the present invention. Total concentration of the surfactant when present may range from 0.1 to 20%, preferably from 1 to 15%, optimally from 1 to 10% by weight of the end use composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan (e.g.Tweens, like Tween 20 and Tween 40) as well as combinations thereof. Alkyl polyglycosides and saccharide fatty amides (e.g. methyl gluconamides) and trialkylamine oxides are also suitable nonionic surfactants.

Suitable anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, C₈-C₂₀ sarcosinates, C₈-C₂₀ acyl lactylates, sulfoacetates and combinations thereof.

Useful amphoteric surfactants include cocoamidopropyl betaine, C₁₂-C₂₀ trialkyl betaines, sodium lauroamphoacetate, and sodium laurodiamphoacetate.

Perfumes may optionally be used in the end use composition of this invention. Illustrative non-limiting examples of the types of perfumes that may be used include those comprising terpenes and terpene derivatives like those described in Bauer, K., et al., Common Fragrance and Flavor Materials, VCH Publishers (1990).

Illustrative yet non-limiting examples of the types of fragrances that may be used in the (end use) compositions of this invention include myrcene, dihydromyrenol, citral, tagetone, cis-geranic acid, citronellic acid, mixtures thereof or the like.

Preferably the amount of fragrance employed in the end use composition of this invention is in the range from 0.0% to 10%, more preferably 0.00001% to 5 wt %, most preferably 0.0001% to 2% of the end use composition.

Sunscreen agents may optionally be included in end use compositions of the present invention. Particularly preferred are such materials as ethylhexyl p-methoxycinnamate, available as Parsol MCX®, Avobenzene, available as Parsol 1789® and benzophenone-3, also known as Oxybenzone. Inorganic sunscreen actives may be employed such as microfine titanium dioxide and zinc oxide. Amounts of the sunscreen agents when present may generally range from 0.0 to 20%, preferably from 0.1 to 12%, optimally from 2 to 8% by weight of the end use composition.

In the event the end use composition comprising the high solvent content emulsion is an antiperspirant or deodorant composition, the same may comprise astringent actives. Examples include aluminum chlorohydrate, aluminum chlorhydrex, aluminum-zirconium chlorhydrex glycine, aluminum sulfate, zinc sulfate, zirconium and aluminum chlorohydroglycinate, zirconium hydroxychloride, zirconium and aluminum lactate, zinc phenolsulfonate and combinations thereof. Amounts of the astringents may range anywhere from 0.5 to 12% by weight of the end use composition.

Oral products formulated with the high solvent content emulsion according to the present invention will optionally contain a fluoride source to prevent dental caries. Typical anti-caries actives include sodium fluoride, stannous fluoride and sodium monofluoro phosphate. Amounts of these materials will be determined by the amount of fluoride releasable which should range between about 500 to about 8800 ppm of the end use composition. Other components of dentifrices can include desensitizing agents such as potassium nitrate and strontium nitrate, sweeteners such as sodium saccharine, aspartame, sucralose, and potassium acesulfam.

Preservatives can desirably be incorporated into the end use compositions of this invention to protect against the growth of potentially harmful microorganisms. Particularly preferred preservatives are phenoxyethanol, methyl paraben, propyl paraben, imidazolidinyl urea, dimethyloldimethylhydantoin, ethylenediaminetetraacetic acid salts (EDTA), sodium dehydroacetate, methylchloroisothiazolinone, methylisothiazolinone, iodopropynbutylcarbamate and benzyl alcohol. The preservatives should be selected having regard for the use of the composition and possible incompatibilities between the preservatives and other ingredients. Preservatives are optionally employed in amounts ranging from 0.01 % to 2% by weight of the end use composition.

End use compositions of the present invention may include vitamins. Illustrative vitamins are Vitamin A (retinol), Vitamin B₂, Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in the end use compositions according to the present invention may range from 0.001 to 10%, preferably from 0.01% to 1%, optimally from 0.1 to 0.5% by weight of the end use composition.

Another type of useful substance for optional use is that of an enzyme such as amylases, oxidases, proteases, lipases and combinations. Particularly preferred is superoxide dismutase, commercially available as Biocell SOD from the Brooks Company, USA. Still other optional additives suitable for use include sunless tanning agents like dihydroxyacetone, antioxidants like lycopene as well as the skin benefit agents like conjugated linoleic acid, and/or petroselinic acid. Even other optional additives include pH modifiers like HCl and NaOH as well as microspheres to scatter light like those made available by Kobo as, for example, MSP-825 and CL-2080.

Skin lightening compounds may optionally be included in the end use compositions of the invention. Illustrative substances are placental extract, lactic acid, vitamin B₃ (niacinamide), arbutin, kojic acid, ferulic acid, resorcinol and derivatives including 4-substituted resorcinols and combinations thereof. Amounts of these agents may range from 0.1 to 10%, preferably from 0.5 to 2% by weight of the end use composition.

Desquamation promoters may optionally be present. Illustrative are the alpha-hydroxycarboxylic acids and beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from about 0.01 to about 15% by weight of the end use composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from green tea, yarrow, chamomile, licorice, aloe vera, grape seed, citrus unshui, willow bark, sage, thyme and rosemary.

Also included may be such materials as lipoic acid, retinoxytrimethylsilane (available from Clariant Corp. under the Silcare 1 M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Amounts of these materials may optionally range from 0.000001 to 10%, preferably from 0.0001 to 1% by weight of the end use composition.

Colorants, opacifiers and abrasives may optionally be included in the end use compositions of the present invention. Each of these substances may range from 0.05 to 5%, preferably between 0.1 and 3% by weight of the end use composition. Colorant often preferred is carbon black.

Especially preferred but optional additives suitable for use in this invention include quaternary ammonium compounds such as 1,2-dihydroxypropyl trimonium chloride as well as derivatives thereof and moisturizing agents such as substituted ureas like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl urea; N-methyl, N'-hydroxyethyl urea; N-ethyl-N'-hydroxyethyl urea; N-hydroxypropyl-N'-hydroxyethyl urea and N,N'dimethyl-N-hydroxyethyl urea or mixtures thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50% aqueous liquid from the National Starch & Chemical Division of ICI under the trademark Hydrovance.

Amounts of optional quaternary ammonium compound and/or substituted urea, when used, in the end use composition of this invention range from 0.01 to 20%, preferably from 0.5 to 15%, and most preferably from 2 to 10% based on total weight of the end use composition and including all ranges subsumed therein.

In a preferred embodiment, the end use composition of this invention comprises at least 50% by weight of high solvent content emulsion, and most preferably 60 to 95% by weight of the high solvent content emulsion. The same is expected to display excellent active deliverability. Furthermore, the emulsion of this invention is suitable to retain high amounts of water and displays a diffusion coefficient of water of less than 1.5 x 10⁻⁹ m/s, preferably less than 1.0 x 10⁻¹⁰ m/s, and most preferably less than 9 x 10⁻¹¹ m/s.

A wide variety of packaging, including biodegradable packaging, can be employed to store and deliver the end use compositions. Packaging is often dependent upon the type of personal care end-use. For instance leave-on skin lotions and creams, shampoos, conditioners and shower gels generally employ plastic containers with an opening at a dispensing end covered by a closure. Typical closures are screw-caps, non-aerosol pumps and flip-top hinged lids. Packaging for antiperspirants, deodorants and depilatories may involve a container with a roll-on ball on a dispensing end. Alternatively these types of personal care products may be delivered in a stick composition formulation in a container with propel-repel mechanism where the stick moves on a platform towards a dispensing orifice. Metallic cans pressurized by a propellant and having a spray nozzle serve as packaging for antiperspirants, shave creams and other personal care products. Toilette bars may have packaging constituted by a cellulosic or plastic wrapper or within a cardboard box or even encompassed by a shrink wrap plastic film.

End use compositions of the present invention, therefore, may be in a variety of forms. These forms may also include mousses, aerosol and non-aerosol sprays and fabric (e.g. non-woven textile)-applied formulations.

The following examples are provided to facilitate an understanding of the present invention. The examples are not intended to limit the scope of the claims.

### Example 1

All samples were made by mixing the mentioned ingredients under conditions of moderate shear, atmospheric pressure and ambient temperature.

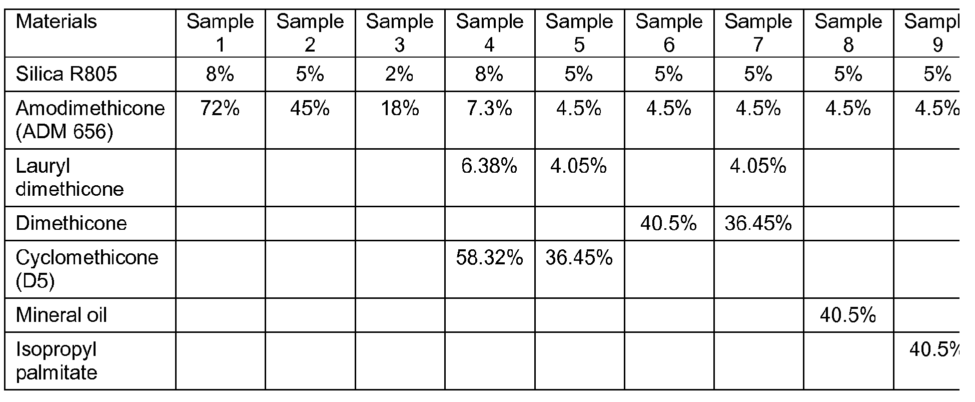

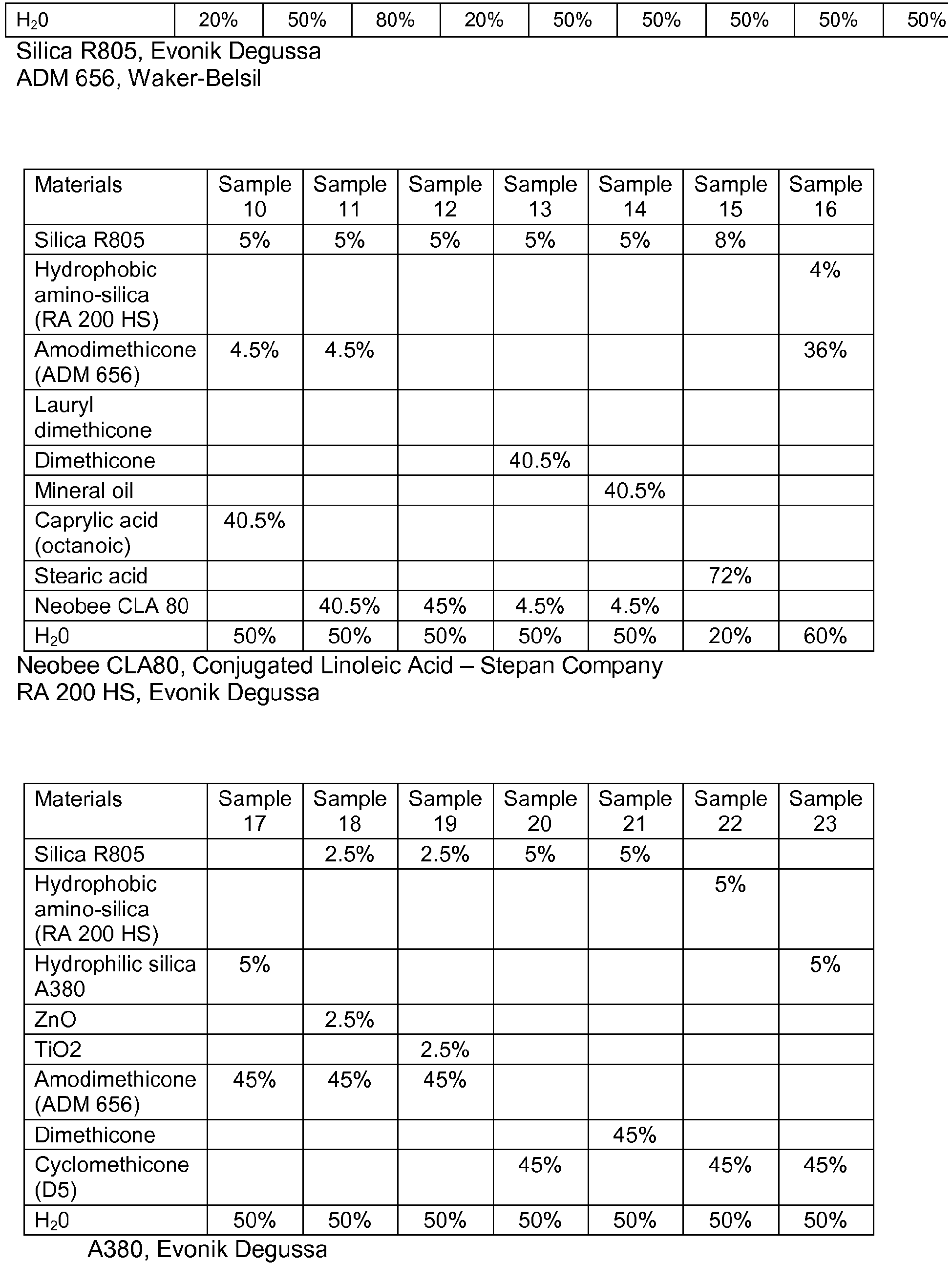

The samples made in this experiment were assessed for appearance and stability. Samples 1-19 displayed an excellent and uniform white appearance. The same samples displayed no syneresis after preparation. Samples 20-23 were not made consistent with this invention. Samples 20-23 were not stable and resulted in phase separation almost immediately after mixing. Coherent anti-Stokes Raman Scattering Spectroscopy revealed that the samples made according to this invention in this experiment were in fact water-in-oil emulsions.

### Example 2

The following were assessed for water diffusion rates.

| Sample | | Water diffusion coefficient (10⁻⁹ m²/s) taken with Magnetic Resonance Imaging T |
|---|---|---|
| 25 | H₂O | 2.0021 |
| 26 | Pickering emulsion: Fumed silica R972(8%)/ cyclomethicone(72%)/H₂O(20%) | 1.605 |
| 27 | Pickering emulsion: Fumed silica R972(5%)/ cyclomethicone(45%)/H₂O(50%) | 1.684 |
| 28 | Silica R805/Amodidimethicone 656/H₂0(20%) | 75.3% at 0.0106, 23.1% at 0.0708 |
| 29 | Silica R805/ Amodidimethicone 656/H₂0(50%) | 92.8% at 0.0716, 6.5% at 0.0000796 |
| 30 | Silica R805/ Amodidimethicone 656/lauryldimethicone/cyclomethicone/H₂0(20%) | 60.1 % at 0.809, 40.2% at 0.0592 |
| 31 | Silica R805/Amodidimethicone 656/lauryldimethicone/cyclomethicone/H₂0(50%) | 91.6% at 1.106, 7.7% at 0.00236 |

| | | |
|---|---|---|
| * Samples 28-31 correspond to samples 1,2,4 and 5 of Example 1, respectively. ** Fumed silica R972 is made available by Evonik Degussa. *** Water diffusion for samples 28-31 shows multiple water diffusion rates observed. Such rates are significantly lower than those observed for conventional Pickering emulsions. The lower water diffusion rates are an indication that the compositions made according to this invention can comprise high water content because water is hydrogen-bonded to the amino group on amodimethicone. T 300MHz Bruker DSX spectrometer with pulsed-field gradient and diffSe (spin echo sequence) and Topspin software. | | |

### Example 3

Water-in-oil and oil-in-water compositions consistent with this invention were made by mixing the ingredients identified below.

**A. Water-in-oil composition**

| Ingredient | Weight % |
|---|---|
| Silica R805 | 2.88 |
| Amodimethicone DC 8500 | 2.60 |
| Silicone volatile DC 245* | 23.36 |
| Water | Balance |
| Cyclopentasiloxane and dimethicone crosspolymer DC 9045 | 38.46 |
| Glycerol | 3.85 |

| | |
|---|---|
| ^{*}made available by Dow Corning | |

**B. Oil-in-water composition**

| Ingredient | Weight % |
|---|---|
| Water | Balance |
| EDTA | 0.05 |
| Phenoxyethanol | 0.40 |
| Carbopol Ultrez 21 (cross-linked acrylate thickener) | 0.40 |
| Xanthum gum | 0.10 |
| NaOH (20%) | 0.42 |
| Methylparaben | 0.20 |
| Propyl paraben | 0.1 |
| Silica R805 | 1.0 |
| Amodimethicone DC 8500 | 0.9 |
| Dimethicone* | 8.1 |
| Glycerol | 20.0 |
| Kobo MSP-825 | 2.0 |

| | |
|---|---|
| * Mixture, 40% with viscosity of 5 centistokes and 60% with viscosity at 50 centistokes, taken at 25°C; DC 200 | |

Assessment of the water-in-oil and oil-in-water compositions made above and consistent with this invention revealed excellent sensory benefits after topical application to skin.

Compositions similar to the water-in-oil composition described above were made except that no particle was used in one set of compositions and no functionalized polymer was used in a second set of compositions. Water was used to make up the balance. The compositions made inconsistent with the compositions of this invention resulted in poor silky sensory characteristics.

Compositions similar to the ones made according to this invention were made with traditional surfactant (e.g. Tween 40, 2% by weight or PEG-10 Dimethicone [KF6017 by Shin Etsu]) in lieu of particle and functionalized polymer. Water was used to make up the balance. The compositions made consistent with this invention had silky sensory characteristics, surprisingly, at least as good as those compositions made with traditional surfactant.

### Example 4

Compositions were prepared by mixing the ingredients below.

| Ingredients | Weight % | Weight % |
|---|---|---|
| Water | Balance | Balance |
| EDTA | 0.05 | 0.05 |
| Phenoxyethanol | 0.4 | 0.4 |
| Carbopol Ultrez 21 (cross-linked acrylate thickener) | 0.4 | 0.4 |
| NaOH (50%) | 0.2 | 0.14 |
| Xanthum gum | 0.1 | 0.1 |
| Tween 40 | 1.0 | 1.0 |
| Glycerol | 5.0 | 30.0 |
| Methylparaben | 0.2 | 0.2 |
| Propyl paraben | 0.1 | 0.1 |
| Silica R805 | 1.0 | 1.0 |
| Amodimethicone DC 8500 | 0.9 | 0.9 |
| Dimethicone* | 8.0 | 8.0 |
| Kobo MSP-825 | 3.0 | 2.0 |

| | | |
|---|---|---|
| * Mixture, 40% viscosity of 5 centistokes and 60% with viscosity at 50 centistokes, taken at 25°C; DC200 | | |

Results demonstrated that compositions made according to this invention may, optionally, be formulated with surfactant. Upon application, panelists concluded that the compositions made according to this example had excellent silky sensory attributes.

## Claims

1. An emulsion comprising:
(a) particles having an isoelectric point below a pH of the emulsion;
(b) a functionalized polymer suitable to hydrogen and/or ionically bond to the particles; and
(c) a solvent;
wherein the particles comprise pyrogenically produced silica, pyrogenically produced Silica comprising 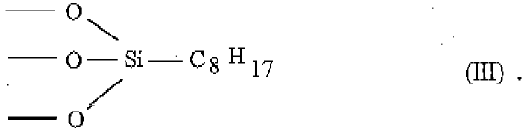 wherein the functionalized polymer is amodimethicone;
wherein the emulsion comprises surfactants in an amount of less than 3% by weight of the emulsion; and
wherein the emulsion comprises at least 20% by weight solvent and the solvent is water.

2. An emulsion according to claim 1 wherein the particles comprise at least 0.1% by weight silica.

3. An emulsion according to claim 1 or claim 2 wherein the particles have a diameter of less than 3 microns.

4. An emulsion according to any one of the preceding claims wherein the functionalized polymer has anionic, zwitterionic and/or cationic character.

5. An emulsion according to claim 4 wherein the functionalized polymer comprises groups with N⁺ and COO⁻.

6. An emulsion according to any one of the preceding claims wherein the emulsion further comprises at least one oil and/or fatty acid.

7. The emulsion according to claim 6 wherein the oil is pentasiloxane, dimethicone and/or mineral oil, and the fatty acid is octanoic acid.

8. An emulsion according to claim 6 or claim 7 wherein the oil makes up from 3 to 85% by weight of the mixture of oil and functionalized polymer.

9. An emulsion according to any one of the preceding claims wherein the emulsion further comprises carbon black.

10. An end use composition comprising the emulsion of claim 1.

11. A composition according to claim 10 wherein the composition further comprises niacinamide, a quaternary ammonium compound or both.

12. A method for treating a skin condition comprising the step of topically applying the end use composition of claim 10 or claim 11.

## Patentansprüche

1. Emulsion, umfassend:
(a) Partikel mit einem isoelektrischen Punkt unter einem pH der Emulsion,
(b) ein funktionalisiertes Polymer, das geeignet ist, über Wasserstoff und/oder ionisch an die Partikel zu binden, und
(c) ein Lösungsmittel,
wobei die Partikel pyrogen hergestelltes Siliciumdioxid umfassen, wobei das pyrogen hergestellte Siliciumdioxid umfasst 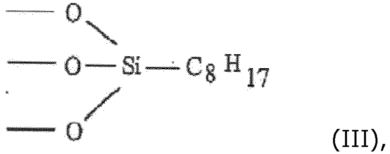 worin das funktionalisierte Polymer Amodimethicon ist,
wobei die Emulsion Tenside in einer Menge von weniger als 3 Gewichts-% der Emulsion umfasst und
wobei die Emulsion mindestens 20 Gewichts-% Lösungsmittel umfasst und das Lösungsmittel Wasser ist.

2. Emulsion nach Anspruch 1, wobei die Partikel mindestens 0,1 Gewichts-% Siliciumdioxid umfassen.

3. Emulsion nach Anspruch 1 oder 2, wobei die Partikel einen Durchmesser von weniger als 3 Mikrometer aufweisen.

4. Emulsion nach irgendeinem der vorhergehenden Ansprüche, wobei das funktionalisierte Polymer anionische, zwitterionische und/oder kationische Eigenschaft zeigt.

5. Emulsion nach Anspruch 4, wobei das funktionalisierte Polymer Gruppen mit N⁺ und COO⁻ umfasst.

6. Emulsion nach irgendeinem der vorhergehenden Ansprüche, wobei die Emulsion des Weiteren mindestens ein Öl und/oder eine Fettsäure umfasst.

7. Emulsion nach Anspruch 6, wobei das Öl Pentasiloxan, Dimethicon und/oder Mineralöl und die Fettsäure Octansäure ist.

8. Emulsion nach Anspruch 6 oder 7, wobei das Öl 3 bis 85 Gewichts-% der Mischung des Öls und des funktionalisierten Polymers ausmacht.

9. Emulsion nach irgendeinem der vorhergehenden Ansprüche, wobei die Emulsion des Weiteren Ruß umfasst.

10. Zusammensetzung zur Endverwendung, umfassend die Emulsion nach Anspruch 1.

11. Zusammensetzung nach Anspruch 10, wobei die Zusammensetzung des Weiteren Niacinamid, eine quaternäre Ammoniumverbindung oder beides umfasst.

12. Verfahren zur Behandlung eines Hautzustands, umfassend den Schritt des aktuellen Einsatzes der Zusammensetzung zur Endverwendung nach Anspruch 10 oder 11.

## Revendications

1. Emulsion comprenant :
(a) des particules présentant un point isoélectrique inférieur à un pH de l'émulsion ;
(b) un polymère fonctionnalisé approprié pour se lier par l'hydrogène et/ou ioniquement aux particules ; et
(c) un solvant ;
dans laquelle les particules comprennent de la silice produite pyrogéniquement, de la silice produite pyrogéniquement comprenant 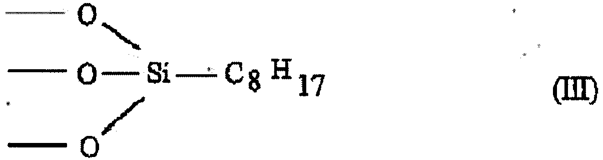 où le polymère fonctionnalisé est l'amodiméthicone ;
où l'émulsion comprend des tensioactifs dans une quantité inférieure à 3 % en masse de l'émulsion ; et
où l'émulsion comprend au moins 20 % en masse de solvant et le solvant est l'eau.

2. Emulsion selon la revendication 1, dans laquelle les particules comprennent au moins 0,1 % en masse de silice.

3. Emulsion selon la revendication 1 ou la revendication 2, dans laquelle les particules présentent un diamètre inférieur à 3 microns.

4. Emulsion selon l'une quelconque des revendications précédentes, dans laquelle le polymère fonctionnalisé présente un caractère anionique, zwitterionique et/ou cationique.

5. Emulsion selon la revendication 4, dans laquelle le polymère fonctionnalisé comprend des groupes avec N⁺ et COO⁻.

6. Emulsion selon l'une quelconque des revendications précédentes, dans laquelle l'émulsion comprend de plus au moins une huile et/ou un acide gras.

7. Emulsion selon la revendication 6, dans laquelle l'huile est le pentasiloxane, la diméthicone et/ou une huile minérale, et l'acide gras est l'acide octanoïque.

8. Emulsion selon la revendication 6 ou la revendication 7, dans laquelle l'huile constitue de 3 à 85 % en masse du mélange d'huile et de polymère fonctionnalisé.

9. Emulsion selon l'une quelconque des revendications précédentes, dans laquelle l'émulsion comprend de plus du noir de carbone.

10. Composition d'utilisation finale comprenant l'émulsion selon la revendication 1.

11. Composition selon la revendication 10, dans laquelle la composition comprend de plus du niacinamide, un composé d'ammonium quaternaire ou les deux.

12. Procédé de traitement d'un état de la peau comprenant l'étape d'application topique de la composition d'utilisation finale selon la revendication 10 ou la revendication 11.
